# EUROPEAN PATENT APPLICATION

(11) **EP 1 279 332 A2**
(43) Date of publication of application: **29.01.2003**
(21) Application number: 02254971.1
(22) Date of filing: 16.07.2002
(51) Int. Cl.: A01K 67/027, C12N 15/54, C12N 15/85, C12N 9/10, A61K 48/00, C12N 5/06, C12N 5/10, A61P 17/06, A61P 43/00, C12Q 1/48

(54) **Transgenic non-human mammal**

(30) Priority: 17.07.2001 GB 0117392
(71) Applicant: Pfizer Limited, Sandwich, Kent CT13 9NJ (GB); PFIZER INC., New York, N.Y. 10017 (US)
(72) Inventor: Burslem, Martyn Frank, Pfizer Global Res. & Devel., Sandwich, Kent CT13 9NJ (GB); Fidock, Mark David, Pfizer Global Res. & Devel., Sandwich, Kent CT13 9NJ (GB)
(74) Representative: Hayles, James Richard

(57) **Abstract**

The invention features transgenic non-human mammals and transgenic cells expressing a radical fringe transgene comprising a radical fringe coding sequence, wherein expression of said coding sequence in said mammal is driven by an operably linked regulatory sequence that directs inducible and keratinocyte-specific expression, and wherein said mammal exhibits accelerated wound healing and/or increased keratinocyte proliferation following the induction of transgene expression. The invention also features polynucleotide (SEQ ID NO: 1) and amino acid (SEQ ID NO: 2) sequences for human radical fringe, methods of treating psoriasis and promoting wound healing by administering a modulator of radical fringe polypeptide activity, and methods of identifying modulators of radical fringe activity.

## Description

### Field of the Invention

The invention features transgenic non-human mammals and transgenic cells expressing a radical fringe transgene comprising a radical fringe coding sequence that is operably linked to a regulatory sequence that directs inducible and keratinocyte-specific expression. These mammals exhibit accelerated wound healing and/or increased keratinocyte proliferation following the induction of transgene expression. The invention also features polynucleotide and amino acid sequences for human radical fringe, methods of treating psoriasis and promoting wound healing, and methods of identifying modulators of radical fringe activity.

### Background

Wound healing is a complex process that involves inflammation, apoptosis, cell proliferation and migration, matrix synthesis, tissue contraction, and tissue remodeling. This process encompasses the interplay between many different tissues, cell lineages, matrix signals, and growth factors. To study the effect of epidermal-specific overexpression of various genes thought to possibly play a role in regulating wound healing, several studies have taken advantage of transgenic models using keratin gene promoters, which drive expression in an epidermal-specific pattern (Bailleul et al., Cell 62: 697-708, 1990; Vassar et al., Proc. Natl. Acad. Sci. USA 86: 1563-67, 1989; Ramirez et al., Differentiation 58: 56-64, 1994).

One particularly useful keratin promoter to use in transgenic models of wound healing is a 2.4 kb fragment of the keratin 6 (K6) promoter. The constitutive expression driven by the full length promoter is absent in this fragment, but it drives inducible expression in keratinocytes following injury, in response to hyperproliferative stimuli such as phorbol 12-myristate 13-acetate (PMA) or all-*trans*-retinoic acid (Ramirez et al., DNA and Cell Biology 17: 177-85, 1998; Larcher et al., Oncogene 17: 303-11, 1998), and in chronic proliferative disorders such as psoriasis (Takahashi and Coulombe, J. Biol. Chem. 272; 11979-85, 1997).

The present invention relates, in part, to the creation of a transgenic mouse expressing the radical fringe gene under the control of the above-described 2.4 kb fragment of the K6 promoter. The radical fringe gene is a member of the mammalian fringe genes that were originally identified based upon their similarity to the Drosophila fringe gene. All of these fringe genes encode glycosyltransferases which participate in the evolutionarily conserved Notch receptor signaling pathway (Yuan et al., Cell 88: 9-11, 1997; Moloney et al., Nature 406: 369-75, 2000). This pathway regulates signal transduction involved in cell fate, tissue organization, and cancer (Artavanis-Tsakonas et al., Science 284: 770-76, 1999; Joutel and Tournier-Lasserve, Semin. Cell Dev. Biol. 9: 619-25, 1998). The human radical fringe gene maps to human Chr17q25 in the minimal region for a familial psoriasis susceptibility locus (Moran et al., Mammalian Genome 10: 535-41, 1999).

### Summary of the Invention

In a first aspect, the invention features a transgenic non-human mammal expressing or capable of expressing a radical fringe transgene comprising a radical fringe coding sequence, wherein expression of the coding sequence in the mammal is driven by an operably linked regulatory sequence that directs inducible and keratinocyte-specific expression, and wherein the mammal exhibits accelerated wound healing and/or increased keratinocyte proliferation following the induction of transgene expression. Preferably, the regulatory sequence comprises the 2.4 kb fragment of the keratin 6 (K6) promoter, the mammal is a rodent, more preferably, a mouse, and/or the radical fringe transgene comprises the murine radical fringe coding sequence disclosed in Genbank Accession No. U94350 or the human radical fringe coding sequence of SEQ ID NO: 1.

In a second aspect, the invention features a transgenic keratinocyte cell expressing or capable of expressing a radical fringe transgene, wherein the transgene comprises a radical fringe coding sequence operably linked to a regulatory sequence that directs inducible and keratinocyte-specific expression, and wherein the keratinocyte exhibits increased keratinocyte proliferation following the induction of transgene expression.

In a third aspect, the invention features a transgenic ES cell comprising a radical fringe transgene, wherein the transgene comprises a radical fringe coding sequence operably linked to a regulatory sequence that directs inducible and keratinocyte-specific expression.

Preferably, the transgenic keratinocyte or ES cell contains a regulatory sequence comprising the 2.4 kb fragment of the keratin 6 (K6) promoter, and/or the radical fringe transgene comprises the murine radical fringe coding sequence disclosed in Genbank Accession No. U94350 or the human radical fringe coding sequence of SEQ ID NO: 1.

Also featured in the invention is an isolated or purified human radical fringe polypeptide comprising the amino acid sequence of SEQ ID NO: 2, or an amino acid sequence encoded by a polynucleotide which will hybridize under highly stringent conditions to the full length complement of SEQ ID NO: 1. In a related aspect, the invention provides an isolated or purified polypeptide comprising an amino acid sequence having at least 95% identity to an amino acid sequence comprising SEQ ID NO: 2.

In another aspect, the invention features an isolated or purified polynucleotide comprising a nucleic acid sequence encoding a human radical fringe polypeptide of SEQ ID NO: 2, the coding sequence shown in SEQ ID NO: 1, or a nucleic acid sequence which hybridizes to the full length of the complement of SEQ ID NO: 1 under highly stringent conditions. In related aspects, the invention provides a vector comprising the above-described polynucleotide and a transgenic host cell comprising the above-described polynucleotide operably linked to a regulatory sequence that drives expression in the host cell or a cell derived from the host cell.

The invention also features a method for producing a human radical fringe polypeptide comprising the amino acid sequence of SEQ ID NO: 2, or an amino acid sequence encoded by a polynucleotide which will hybridize under highly stringent conditions to the full length complement of SEQ ID NO: 1, the method comprising culturing a transgenic host cell under conditions suitable for expression of the polypeptide, wherein said cell comprises a nucleic acid sequence encoding a human radical fringe polypeptide comprising the amino acid sequence of SEQ ID NO: 2, the coding sequence shown in SEQ ID NO: 1, or a nucleic acid sequence which hybridizes to the full length of the complement of SEQ ID NO: 1 under highly stringent conditions, and wherein the polynucleotide is operably linked to a regulatory sequence that drives expression in said host cell.

In other aspects, the invention provides a method of treating or preventing psoriasis in a patient comprising administering an agent that reduces radical fringe activity in the epidermis of said patient, and a method of promoting wound healing comprising administering an agent that increases radical fringe activity in the epidermis of the patient.

The invention also features a method of identifying an agent that modulates radical fringe activity comprising contacting an agent with the human radical fringe polypeptide comprising the amino acid sequence of SEQ ID NO: 2, or an amino acid sequence encoded by a polynucleotide which will hybridize under highly stringent conditions to the full length complement of SEQ ID NO: 1, and measuring radical fringe activity, wherein a difference between the radical fringe activity in the presence of the agent and in the absence of the agent is indicative that the agent modulates the activity.

In another aspect, the invention provides a method of identifying an agent that modulates radical fringe activity comprising contacting an agent with the transgenic host cell comprising a polynucleotide encoding a human radical fringe polypeptide comprising the amino acid sequence of SEQ ID NO: 2, the coding sequence shown in SEQ ID NO: 1, or a nucleic acid sequence which hybridizes to the full length of the complement of SEQ ID NO: 1 under highly stringent conditions, wherein the polynucleotide is operably linked to a regulatory sequence that drives expression in the host cell, and measuring radical fringe activity, wherein a difference between said radical fringe activity in the presence of the agent and in the absence of the agent is indicative that the agent modulates said activity.

The invention also features a method of identifying an agent that modulates radical fringe activity comprising inducing transgene expression in a transgenic non-human mammal capable of expressing a radical fringe transgene comprising a radical fringe coding sequence operably linked to a regulatory sequence that directs inducible and keratinocyte-specific expression or a transgenic keratinocyte cell capable of expressing a radical fringe transgene, wherein the transgene comprises a radical fringe coding sequence, wherein expression of the coding sequence in the keratinocyte is driven by an operably linked regulatory sequence that directs inducible and keratinocyte-specific expression, contacting the agent with the mammal or cell, and measuring radical fringe activity, wherein a difference in the radical fringe activity in the presence of the agent and in the absence of the agent is indicative that the agent modulates the activity.

The invention also features the agents identified by the methods described above.

Another embodiment of the invention is the use of agents that reduce radical fringe activity for the treatment of psoriasis. The invention also features the use of agents that increase radical fringe activity for the treatment of wounds, including acute wounds such as surgical wounds, age-impaired wounds, chronic ulcers, ulcers caused by venous stasis, ischemic ulcers, diabetic neuropathic ulcers, pressure sores etc.

Those skilled in the art will fully understand the terms used herein in the description and the appendant claims to describe the present invention. Nonetheless, unless otherwise provided herein, the following terms are as described immediately below.

An "agent that increases radical fringe activity" refers to a molecule which intensifies or mimics the biological activity of a radical fringe polypeptide. Such agents (i.e., agonists) may include proteins, nucleic acids, carbohydrates, small molecules, or any other compound or composition which increases the activity of a radical fringe either by increasing the amount of radical fringe present in a cell or by increasing the signaling of a radical fringe polypeptide in its signal transduction pathway.

An "agent that decreases radical fringe activity" refers to a molecule which inhibits or attenuates the biological activity of a radical fringe polypeptide. Such agents (i.e., antagonists) may include proteins such as anti-radical fringe antibodies, nucleic acids, carbohydrates, small molecules, or any other compound or composition which decreases the activity of a radical fringe polypeptide either by reducing the amount of radical fringe polypeptide present in a cell, or by decreasing the signaling of a radical fringe polypeptide in its signal transduction pathway.

An "allelic variant" is an alternative form of the gene encoding a radical fringe. Allelic variants may result from at least one mutation in the nucleic acid sequence and may result in altered mRNAs or in polypeptides whose structure or function may or may not be altered. A gene may have none, one, or many allelic variants of its naturally occurring form. Common mutational changes which give rise to allelic variants are generally ascribed to naturally occurring deletions, additions, or substitutions of nucleotides. Each of these types of changes may occur alone, or in combination with the others, one or more times in a given sequence.

"Altered" nucleic acid sequences encoding a radical fringe polypeptide include those sequences with deletions, insertions, or substitutions of different nucleotides, resulting in a polypeptide with at least one functional characteristic of a radical fringe polypeptide. Included within this definition are polymorphisms which may or may not be readily detectable using a particular oligonucleotide probe of the polynucleotide encoding a radical fringe polypeptide. The encoded protein may also be "altered," and may contain deletions, insertions, or substitutions of amino acid residues which produce a silent change and result in a functionally equivalent radical fringe polypeptide. Deliberate amino acid substitutions may be made on the basis of similarity in polarity, charge, solubility, hydrophobicity, hydrophilicity, and/or the amphipathic nature of the residues, as long as the biological or immunological activity of the radical fringe polypeptide is retained. For example, negatively charged amino acids may include aspartic acid and glutamic acid, and positively charged amino acids may include lysine and arginine. Amino acids with uncharged polar side chains having similar hydrophilicity values may include: asparagine and glutamine; and serine and threonine. Amino acids with uncharged side chains having similar hydrophilicity values may include: leucine, isoleucine, and valine; glycine and alanine; and phenylalanine and tyrosine.

"Amplification" relates to the production of additional copies of a nucleic acid sequence. It is generally carried out using polymerase chain reaction (PCR) technologies well known in the art.

A "composition" comprising a given polynucleotide or amino acid sequence refers broadly to any composition containing the given polynucleotide or amino acid sequence. The composition may comprise a dry formulation or an aqueous solution.

"Conservative amino acid substitutions" are those substitutions that, when made, least interfere with the properties of the original protein, i.e., the structure and especially the function of the protein is conserved and not significantly changed by such substitutions. Examples of conservative amino acid substitutions include the following: Ala replaced with Gly or Ser; Arg replaced with His or Lys; Asn replaced with Asp, Gln, or His; Asp replaced with Asn or Glu; Cys replaced with Ala or Ser; Gln replaced with Asn, Glu, or His; Glu replaced with Asp, Gin, or His; Gly replaced with Ala; His replaced with Asn, Arg, Gln, or Glu; lie replaced with Leu or Val; Leu replaced with lie or Val; Lys replaced with Arg, Gln, or Glu; Met replaced with Leu or Ile; Phe replaced with His, Met, Leu, Trp, or Tyr; Ser replaced with Cys or Thr; Thr replaced with Ser or Val; Trp replaced with Phe or Tyr; Tyr replaced with His, Phe, or Trp; and Val replaced with lle, Leu, or Thr. Conservative amino acid substitutions generally maintain (a) the structure of the polypeptide backbone in the area of the substitution, for example, as a beta sheet or alpha helical conformation, (b) the charge or hydrophobicity of the molecule at the site of the substitution, and/or (c) the bulk of the side chain.

The term "derivative" refers to the chemical modification of a polypeptide sequence, or a polynucleotide sequence. Chemical modifications of a polynucleotide sequence can include, for example, replacement of hydrogen by an alkyl, acyl, hydroxyl, or amino group. A derivative polynucleotide encodes a polypeptide which retains at least one biological or immunological function of the natural molecule. A derivative polypeptide is one modified by glycosylation, pegylation, or any similar process that retains at least one biological or immunological function of the polypeptide from which it was derived.

A "fragment" is a unique portion of a radical fringe polypeptide or the polynucleotide encoding a radical fringe polypeptide which is identical in sequence to but shorter in length than the parent sequence. A fragment used as a probe, primer, antigen, therapeutic molecule, or for other purposes, may be at least 5, 10, 15, 20, 25, 30, 40, 50, 60, 75, 100, 150, 250 or at least 500 contiguous nucleotides or amino acid residues in length. Fragments may be preferentially selected from, or lack, certain regions of a molecule.

By a "2.4 kb fragment of the keratin 6 (K6) promoter" is meant the 2.4 kb promoter fragment described in Ramirez et al., DNA and Cell Biology 17: 177-85, 1998, and Larcher et al., Oncogene 17: 303-11, 1998 that drives keratinocyte-specific expression in an inducible manner.

The term "identity" refers to a degree of complementarity. There may be partial similarity or complete identity. The word "similarity" may substitute for the word "identity." A partially complementary sequence that at least partially inhibits an identical sequence from hybridizing to a target nucleic acid is referred to as "substantially similar." The inhibition of hybridization of the completely complementary sequence to the target sequence may be examined using a hybridization assay (Southern or Northern blot, solution hybridization, and the like) under conditions of reduced stringency. A substantially similar sequence or hybridization probe will compete for and inhibit the binding of a completely similar (identical) sequence to the target sequence under conditions of reduced stringency. This is not to say that conditions of reduced stringency are such that non-specific binding is permitted. Rather, reduced stringency conditions require that the binding of two sequences to one another be a specific (i.e., a selective) interaction. The absence of non-specific binding may be tested by the use of a second target sequence which lacks even a partial degree of complementarity (e.g., less than about 30% similarity or identity). In the absence of non-specific binding, the substantially similar sequence or probe will not hybridize to the second non-complementary target sequence.

The phrases "percent identity" and "% identity," as applied to polynucleotide sequences, refer to the percentage of residue matches between at least two polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Percent identity between polynucleotide sequences may be determined using the default parameters of the CLUSTAL V algorithm as incorporated into the MEGALIGN version 3.12e sequence alignment program. This program is part of the LASERGENE software package, a suite of molecular biological analysis programs (DNASTAR, Madison, WI). CLUSTAL V is described in Higgins and Sharp, CABIOS 5:151-153, 1989 and in Higgins et al., CABIOS 8:189-19, 1992. For pairwise alignments of polynucleotide sequences, the default parameters are set as follows: Ktuple=2, gap penalty=5, window=4, and "diagonals saved"=4. The "weighted" residue weight table is selected as the default. Percent identity is reported by CLUSTAL V as the "percent similarity" between aligned polynucleotide sequence pairs. Alternatively, a suite of commonly used and freely available sequence comparison algorithms is provided by the National Center for Biotechnology Information (NCBI) Basic Local Alignment Search Tool (BLAST) (Altschul et al., J. Mol. Biol. 215:403- 410, 1990), which is available from several sources, including the NCBI, Bethesda, MD, and at http://www.ncbi.nlm.nih.gov/BLAST/. The BLAST software suite includes various sequence analysis programs including "blastn," that is used to align a known polynucleotide sequence with other polynucleotide sequences from a variety of databases. Also available is a tool called "BLAST 2 Sequences" that is used for direct pairwise comparison of two nucleotide sequences. "BLAST 2 Sequences" can be accessed and used interactively at http://www.ncbi.nlm.nih.gov/blast/bl2seq/bl2.html. The "BLAST 2 Sequences" tool can be used for both blastn and blastp (discussed below). BLAST programs are commonly used with gap and other parameters set to default settings. For example, to compare two nucleotide sequences, one may use blastn with the "BLAST 2 Sequences" tool Version 2.0.9 (May-07-1999) set at default parameters. Such default parameters may be, for example Matrix: BLOSUM62 Rewardfor match: 1 Penaltyfor mismatch: -2 Open Gap: 5 and Extension Gap.- 2 penalties Gap x drop-off.- 50 Expect: 10 Word Size: 1 Filter: on. Percent identity may be measured over the length of an entire defined sequence, for example, as defined by a particular SEQ ID number, or may be measured over a shorter length, for example, over the length of a fragment taken from a larger, defined sequence, for instance, a fragment of at least 20, at least 30, at least 40, at least 50, at least 70, at least 100, or at least 200 contiguous nucleotides. Such lengths are exemplary only, and it is understood that any fragment length disclosed by the sequences shown herein may be used to describe a length over which percentage identity may be measured. Nucleic acid sequences that do not show a high degree of identity may nevertheless encode similar amino acid sequences due to the degeneracy of the genetic code. It is understood that changes in a nucleic acid sequence can be made using this degeneracy to produce multiple nucleic acid sequences encompassed by the invention that all encode substantially the same radical fringe polypeptide.

The phrases "percent identity" and "% identity," as applied to polypeptide sequences, refer to the percentage of residue matches between at least two polypeptide sequences aligned using a standardized algorithm. Methods of polypeptide sequence alignment are well known. Some alignment methods take into account conservative amino acid substitutions. Such conservative substitutions, explained in more detail above, generally preserve the hydrophobicity and acidity at the site of substitution, thus preserving the structure and function of the polypeptide. Percent identity between polypeptide sequences may be determined using the default parameters of the CLUSTAL V algorithm as incorporated into the MegAlign® sequence alignment program (DNASTAR, Madison, WI). For pairwise alignments of polypeptide sequences using CLUSTAL V, the default parameters are set as follows: Ktuple=I, gap penalty-3, window-5, and "diagonals saved"=5. The PAM250 matrix is selected as the default residue weight table. As with polynucleotide alignments, the percent identity is reported by CLUSTAL V as the "percent similarity" between aligned polypeptide sequence pairs.

Alternatively, the NCBI BLAST software suite may be used. For example, for a pairwise comparison of two polypeptide sequences, one may use the "BLAST 2 Sequences" tool Version 2.0.9 (May-07-1999) with blastp set at default parameters. Such default parameters may be, for example, Matrix: BLOSUM62 Open Gap: 11 and Extension Gap: 1 penalties; Gap x drop-off: 50; Expect: 10 Word Size: 3 Filter: on. Percent identity may be measured over the length of an entire defined polypeptide sequence, for example, as defined by a particular SEQ ID number (e.g., 2, 4, 6, 8, or 10), or may be measured over a shorter length, for example, over the length of a fragment taken from a larger, defined polypeptide sequence, for instance, a fragment of at least 15, at least 20, at least 30, at least 40, at least 50, at least 70 or at least 100 contiguous residues. Such lengths are exemplary only, and it is understood that any fragment length supported by the sequences shown herein, including the Figures and Sequence Listing, may be used to describe a length over which percentage identity may be measured.

By a "host" is meant a transgenic cell (e.g., mammalian, bacterial, insect) or an animal (e.g., non-human mammal) that is transfected with, and capable of expressing, a heterologous polynucleotide.

A "heterologous" polynucleotide is one which is foreign, or non-naturally occurring, or non-naturally positioned in the genome of the host cell.

"Hybridization" refers to the process by which a polynucleotide strand anneals with a complementary strand through base pairing under defined hybridization conditions. Specific hybridization is an indication that two nucleic acid sequences share a high degree of identity. Specific hybridization complexes form under permissive annealing conditions and remain hybridized after the "washing" step(s). The washing step(s) is particularly important in determining the stringency of the hybridization process, with more stringent conditions allowing less non-specific binding, i.e., binding between pairs of nucleic acid strands that are not perfectly matched. Permissive conditions for annealing of nucleic acid sequences are routinely determinable by one of ordinary skill in the art and may be consistent among hybridization experiments, whereas wash conditions may be varied among experiments to achieve the desired stringency, and therefore hybridization specificity. Permissive annealing conditions occur, for example, at 68ºC in the presence of about 6X SSC, about 1% (w/v) SDS, and about 100 pg/ml denatured salmon sperm DNA.

Generally, stringency of hybridization is expressed, in part, with reference to the temperature under which the wash step is carried out. Generally, such wash temperatures are selected to be about 5ºC to 20ºC lower than the thermal melting point (Tm) for the specific sequence at a defined ionic strength and pH. The Tm is the temperature (under defined ionic strength and pH) at which 50% of the target sequence hybridizes to a perfectly matched probe. An equation for calculating Tm and conditions for nucleic acid hybridization are well known and can be found in Sambrook et al., 1989, *Molecular Cloning: A Laboratory Manual,* 2^{nd} ed., Vol. 1-3, Cold Spring Harbor Press, Plainview, NY; specifically see Vol. 2, chapter 9.

High stringency conditions for hybridization between polynucleotides of the present invention include wash conditions of about 55-68ºC in the presence of about 0.2-1.0X SSC and about 0.1% SDS, for 1 hour.

In general, hybridization reactions can be carried out at temperatures of about 65ºC, 60ºC, 55ºC, or 42ºC. SSC concentration may be varied from about 0.1 to 2X SSC, with SDS being present at about 0.1%. Typically, blocking reagents are used to block non-specific hybridization. Such blocking reagents include, for instance, denatured salmon sperm DNA at about 100-200 pg/ml. Organic solvent, such as formamide at a concentration of about 35-50% v/v, may also be used under particular circumstances, such as for RNA:DNA hybridizations. Useful variations on these wash conditions will be readily apparent to those of ordinary skill in the art. Hybridization, particularly under high stringency conditions, is suggestive of evolutionary similarity between the nucleotides. Such similarity is strongly indicative of a similar role for the nucleotides and their encoded polypeptides.

The term "hybridization complex" refers to a complex formed between two nucleic acid sequences by virtue of the formation of hydrogen bonds between complementary bases. A hybridization complex may be formed between sequences present in solution or formed between one nucleic acid sequence present in solution and another nucleic acid sequence immobilized on a solid support (e.g., paper, membranes, filters, chips, pins or glass slides).

By "isolated or purified" is meant changed from the natural state "by the hand of man." If a polynucleotide or polypeptide exists in nature, then it is "isolated or purified" if it is changed and/or removed from its original environment. For example, an "isolated or purified" polynucleotide is separated from other polynucleotide sequence with which it is associated in nature. For example, a cDNA sequence that is removed from intronic sequence normally associated with the coding sequence is "isolated or purified." An "isolated or purified" polynucleotide sequence may be introduced into host cells in culture or in whole organisms and still be "isolated and purified," because the polynucleotide would not be in its naturally occurring form or environment. However, polynucleotide sequences as found in cDNA libraries are excluded from what is meant by "isolated or purified." An "isolated or purified" polypeptide is separated from at least one cellular component with which it is associated in nature. Preferably, the polypeptide is at least 60% free, more preferably, at least 75% free, and, most preferably, at least 90% from other components.

"Operably linked" refers to the situation in which a first nucleic acid sequence is placed in a functional relationship with a second nucleic acid sequence. For example, a promoter is operably linked to a coding sequence if the promoter functions to regulate transcription or expression of the coding sequence. Generally, operably linked DNA sequences may be in close proximity or contiguous and, where necessary to join two protein coding regions, in the same reading frame.

"Polynucleotide" generally refers to any RNA (e.g., mRNA), RNA-like, DNA (e.g., cDNA or genomic), or DNA like sequences, including, without limit, single-stranded, double-stranded, and triple-stranded sequence, sense or antisense strands, sequence generated using nucleotide analogs, hybrid molecules comprising RNA and DNA, and RNA or DNA containing modified bases. The polynucleotide can be naturally-occurring or synthesized.

The term "polypeptide" refers to an amino acid sequence, oligopeptide, peptide, polypeptide, or protein sequence, or a fragment of any of these, and to naturally occurring or synthetic molecules. It includes amino acid sequences modified either by natural processes, such as post-translational processing, or by chemical modifications well known in the art (see, e.g., *Proteins - Structure and Molecular Properties,* Ed. Creighton, W.H. Freeman and Co., New York, NY, 2^{nd} Ed, 1993; *Posttranslational Covalent Modification of Proteins,* Ed. Johnson, Academic Press, New York, NY, 1983; Seifter et al., Meth. Enzymol., 182: 626-46, 1990; and Rattan et al., Ann. NY Acad. Sci. 663: 48-62, 1992).

By "radical fringe activity" is meant radical fringe-specific glycosyltransferase activity *in vitro, in vivo,* or *in situ* radical fringe-specific glycosylation of the Notch receptor, radical fringe-specific keratinocyte proliferation, or radical fringe-specific wound healing.

A "substitution" refers to the replacement of one or more amino acids or nucleotides by different amino acids or nucleotides, respectively.

A "variant" of a particular nucleic acid sequence is defined as a nucleic acid sequence having at least 40% sequence identity to the particular nucleic acid sequence over a certain length of one of the nucleic acid sequences using blastn with the "BLAST 2 Sequences" tool Version 2.0.9, set at default parameters. Such a pair of nucleic acids may show, for example, at least 50%, at least 60%, at least 70%, at least 80%, at least 85%, at least 90%, at least 95%, or at least 98%, or greater, sequence identity over a certain defined length. A variant may be described as, for example, an "allelic" (as defined above), "splice," "species," or "polymorphic" variant. A splice variant may have significant identity to a reference molecule, but will generally have a greater or lesser number of polynucleotides due to alternate splicing of exons during mRNA processing. The corresponding polypeptide may possess additional functional domains or lack domains that are present in the reference molecule. Species variants are polynucleotide sequences that vary from one species to another. The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs) in which the polynucleotide sequence varies by one nucleotide base. The presence of SNPs may be indicative of, for example, a certain population, a disease state, or a propensity for a disease state.

"Transformation" or "transfection" describes a process of genetic modification by which heterologous DNA enters and renders a recipient cell capable of expressing the heterologous DNA. Transformation may occur in a prokaryotic or eukaryotic host cell according to various methods well known in the art. The method is selected based on the type of host cell being transformed and includes, but is not limited to, viral infection, electroporation, heat shock, lipofection, and particle bombardment. The terms "transformed cells" or "transfected cells" include stably transformed cells in which the inserted DNA is capable of replication either as an autonomously replicating plasmid or as part of the host chromosome, as well as transiently transformed or transfected cells which express the inserted DNA or RNA for limited periods of time. All of such transformed or transfected cells are referred to as "transgenic."

By a "transgenic non-human mammal" is meant a non-human mammal containing a heterologous transgene present as an extrachromosomal element or, preferably, present as a stably integrated element present in the chromosomal DNA of the mammal. Vectors for stable integration into the genome of a cell include plasmids, viruses, including retroviruses, and artificial chromosomes. The transgenic non-human mammal of the invention is originally produced, for example, by creating a blastocyst or embryo carrying the desired transgene and then implanting the blastocyst or embryo in a pseudopregnant foster mother for *in utero* development. A preferred method of making the transgenic blastocyst or embryo is by zygote injection in which DNA containing the desired transgene is injected into a fertilized egg or zygote. The transgenic blastocyst or embryo can also be made by first genetically-modifying an embryonic stem (ES) cell and then implanting the ES cell into a blastocyst or aggregating the ES cell with tetraploid embryos. Alternatively, various species of genetically-modified embryos can be obtained by nuclear transfer. In the case of nuclear transfer, the donor cell is a somatic cell or a pluripotent stem cell, and it is engineered to contain the desired transgene. The nucleus of this cell is then transferred into a fertilized or parthenogenetic oocyte that is enucleated, the embryo is reconstituted, and developed into a blastocyst. A genetically-modified blastocyst produced by any of the above methods is then implanted into a pseudopregnant foster mother according to standard methods well known to those skilled in the art. A "transgenic non-human mammal" includes all progeny of the mammal created by the methods described above, provided that the progeny inherit at least one copy of the transgene. It is preferred that all somatic cells and germline cells of the transgenic mammal contain the modification. Preferred transgenic non-human mammals of the invention include rodents, such as mice and rats, cats, dogs, rabbits, guinea pigs, hamsters, sheep, pigs, and ferrets.

By a "pseudopregnant" foster mother or female is meant a female in estrous who has mated with a vasectomized male; she is competent to receive embryos but does not contain any of her own endogenous fertilized eggs.

By a "transgenic animal cell" is meant an animal cell, including a human cell, created by genetic engineering to contain the desired transgene, as well as daughter cells that inherit the transgene. These cells may be genetically-modified in culture according to any standard method known in the art. As an alternative to genetically modifying the cells in culture, non-human mammalian cells may also be isolated from a transgenic non-human mammal that contains the desired transgene. The animal cells of the invention may be obtained from primary cell or tissue preparations as well as culture-adapted, tumorigenic, or transformed cell lines. The preferred genetically-modified cells are ES cells, more preferably, mouse or rat ES cells, and, most preferably, human ES cells. In an alternative embodiment, the preferred transgenic cells are keratinocytes.

By an "ES cell" is meant a pluripotent stem cell derived from an embryo, from a primordial germ cell, or from a teratocarcinoma, that is capable of indefinite self renewal as well as differentiation into cell types that are representative of all three embryonic germ layers.

By "modulates" is meant increases or decreases (including a complete elimination).

Other features and advantages of the invention will be apparent from the following detailed description and from the claims. While the invention is described in connection with specific embodiments, it will be understood that other changes and modifications that may be practiced are also part of this invention and are also within the scope of the appendant claims. This application is intended to cover any equivalents, variations, uses, or adaptations of the invention that follow, in general, the principles of the invention, including departures from the present disclosure that come within known or customary practice within the art, and that are able to be ascertained without undue experimentation. Additional guidance with respect to making and using nucleic acids and polypeptides is found in standard textbooks of molecular biology, protein science, and immunology (see, e.g., Davis et al., *Basic Methods in Molecular Biology,* Elsevir Sciences Publishing, Inc., New York, NY,1986; Hames et al., *Nucleic Acid Hybridization,* IL Press, 1985; *Molecular Cloning,* Sambrook et al., *Current Protocols in Molecular Biology,* Eds. Ausubel et al., John Wiley and Sons; *Current Protocols in Human Genetics,* Eds. Dracopoli et al., John Wiley and Sons; *Current Protocols in Protein Science,* Eds. John E. Coligan et al., John Wiley and Sons; and *Current Protocols in Immunology*, Eds. John E. Coligan et al., John Wiley and Sons). All publications mentioned herein are incorporated by reference in their entireties.

### Description of the Figures

Figure 1A shows the human radical fringe nucleotide coding sequence (SEQ ID NO: 1). Figure 1B shows the predicted amino acid sequence for the human radical fringe polypeptide.

Figure 2 contains graphs showing the kinetics of wound healing in transgenic mice expressing radical fringe under the regulatory control of the 2.4 kb fragment of the K6 promoter. Transgenic line 32645 (gray, n=9) and line 32654 (white, n=9) were compared to controls (black, n=43). Wound area on the day of injury was designated 100%. Figure 2A shows the percent animals with greater than 25% healing, Figure 2B shows the percent animals with greater than 50% healing, and Figure 2C shows the percent animals with greater than 75% healing.

Figure 3 shows skin sections from 5-day old wounds from control (A) and radical fringe transgenic (B) mice. Wound epithelialization is complete in the transgenic mouse but not the control mouse.

Figure 4 shows sections from the PMA treated ears of control (A) and radical fringe transgenic (B) mice, demonstrating the increased keratinocyte proliferation in the ears of the transgenic mice.

### Detailed Description

### The Radical Fringe Transgene

The transgenic non-human mammals and animal cells of the invention contain a radical fringe coding sequence operably linked to a promoter that drives expression in keratinocytes in an inducible fashion. In one preferred embodiment, the promoter is the 2.4 kb fragment of the K6 promoter that drives expression in a keratinocyte-specific pattern following induction with a hyperproliferation or injury stimulus (Ramirez et al., DNA and Cell Biology 17: 177-85, 1998; Larcher et al., Oncogene 17: 303-11, 1998). Alternatively, a promoter can be designed to contain the epidermal growth factor (EGF) response element of a keratin promoter (to achieve keratinocyte-specific expression) coupled with a scheme that provides for a conditional or inducible expression pattern according to standard methods known in the art.

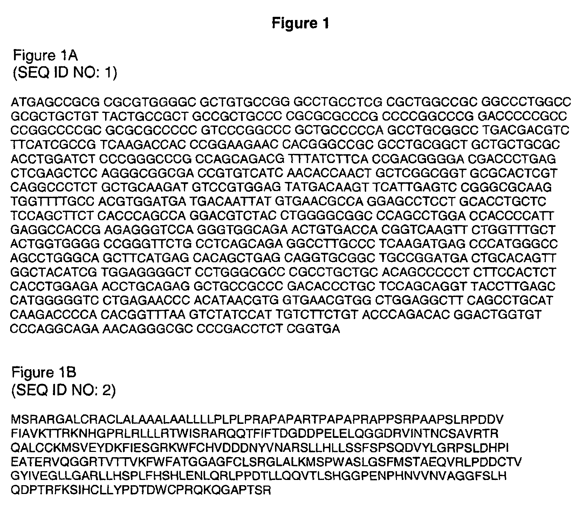

The radical fringe coding sequence contained in the transgene is a vertebrate radical fringe coding sequence, such as the murine sequence shown in Genbank Accession No. U94350, or the human sequence shown in Figure 1A and described below. Typically, the transgene DNA is inserted into an expression vector (e.g., a plasmid or viral vector) and expressed in a yeast, mammalian, or bacterial expression system. For the generation of transgenic cells using microinjection, the transgene is first excised from the vector and then purified.

### Transgenic Non-human Mammals and Animal Cells

The transgenic non-human mammals of the invention express the above-described radical fringe transgene. There are a number of techniques which permit the introduction of the radical fringe transgene into an embryo that is then developed into a transgenic non-human mammal. The most commonly used protocol involves the direct injection of the radical fringe transgene into the pronucleus of a fertilized egg, preferably a male fertilized egg (Hogan et al., *Manipulating the Mouse Embryo (A Laboratory Manual),* Cold Spring Harbor Laboratory Press, Cold Spring Harbor, NY, 1994, 2^{nd} edition, Breban et al., J. Immunol. 156: 794, 1996; Gariepy et al., J. Clin. Invest. 102: 1092, 1998), or into a cell of a zygote. The genetically-modified embryos that develop by this process may have one or several copies of the transgene integrated into their genomes, usually in one integration site.

An alternative method to introduce the transgene into a non-human mammalian embryo involves transfecting ES cells such that they contain the radical fringe transgene according to methods known in the art, such as electroporation, infection with retroviral vectors, or lipofection, and then implanting the ES cells into suitable blastocyst hosts (Capecchi, Trends Genet. 5: 70; 1989) or associating the ES cell with a tetraploid embryo. This technique is especially successful for transfection with very large human artificial chromosomes (HACs), bacterial artificial chromosomes (BACs), or yeast artificial chromosomes (YACs) (Hodgson et al., Neuron 23: 181, 1999; Lamb et al., Nature Neuroscience 2: 695, 1999). Due to positional effects, expression of a randomly integrated transgene may be inhibited or occur in a non-authentic manner with respect to the chosen promoter. To overcome these potential problems, transgenes can be inserted into predetermined loci (e.g., ROSA26 or HPRT) that support transcriptional activity without adverse effects (Zambrowicz et al., Proc. Natl. Acad. Sci. USA 94: 3789, 1997; Soriano et al., Nature Genetics 21: 70, 1999).

Regardless of how the genetically modified embryos or blastocysts are created, they are then implanted in pseudopregnant female mice and allowed to develop *in utero* (Hogan et al., 1994, *supra; Teratocarcinomas and Embryonic Stem Cells: A Practical Approach,* E.J. Robertson, ed., IRL Press, Washington, D.C., 1987).

The transgenic offspring born to the pseudopregnant females are termed "founder" animals. The animals are tested to ensure the desired genotypic change has occurred. This can be done, for example, by detecting the presence of the transgene by PCR with specific primers, or by Southern blotting of tail DNA with a transgene-specific probe. Once the desired genotype has been confirmed, the transgenic non-human mammals are bred to establish transgenic lines and then backcrossed into the genetic background of choice. It is convenient to have the transgene insertion on both chromosomes as this eliminates the need for repeated genotyping in the course of routine animal husbandry.

Once the desired genotype has been confirmed, the transgenic line is subjected to various tests to determine whether the desired gain-of-function phenotype is present. The transgenic non-human mammals of the invention have a sufficient level of expression of the radical fringe transgene to result in accelerated wound healing and an increased keratinocyte proliferation following a hyperproliferation or injury-induced stimulus. These phenotypes can be determined by any method known in the art, such as PMA treatment, 12-O-tetradecanoylphorbol-13-acetate (TPA) treatment, and injury induction protocols, as described in the art (Takahashi et al., J. Biol. Chem. 272: 11979-85, 1997; Schweizer et al., J. Invest. Dermatol. 89: 125-31, 1987; and Fowlis et al., Cell Growth Diff. 7: 679-87, 1996), and in Example 1 below.

Transgenic keratinocytes can be transformed to express the radical fringe transgene or can be derived as primary keratinocytes from the above-described transgenic non-human mammals according to methods known in the art (Ramirez et al., Proc. Natl. Acad. Sci. USA: 92: 4783-87, 1995; Fowlis et al., Cell Growth and Diff. 7: 679-87, 1996).

### Therapeutic Applications

The phenotype of the transgenic non-human mammals of the invention, i.e., the hyperproliferative response of keratinocytes, following stimuli such as PMA or injury, demonstrates that transgenic non-human mammals and keratinocytes expressing the radical fringe transgene are useful as models of psoriasis. Thus, these transgenic non-human mammals and keratinocytes are useful tools in screening assays to identify agents that reduce radical fringe activity. Such agents include those demonstrated to decrease radical fringe-associated signalling and to decrease radical fringe expression. These agents can be useful as therapeutics for treating or preventing disorders associated with hyperproliferation in the skin, such as psoriasis.

In addition, the enhanced wound healing demonstrated in the transgenic non-human mammals of the invention also indicates that agents that increase radical fringe activity are useful as agents to induce or accelerate wound healing for many different wounds, e.g. acute wounds such as surgical wounds, age-impaired wounds, chronic ulcers, ulcers caused by venous stasis, ischemic ulcers, diabetic neuropathic ulcers, pressure sores etc.. Such agents include those demonstrated to increase radical fringe-associated signalling, or to increase radical fringe gene expression. In addition, compositions that contain a radical fringe protein, and compositions that contain cells that allow for *ex vivo* expression of radical fringe protein in a patient, can also be administered to promote wound healing.

Topical administration is the preferred route for the administration of any agent or composition that modulates or mimics radical fringe activity.

### Screening Assays

Agents that modulate radical fringe activity, for example, for use as treatments for psoriasis or wound healing, can be identified by screening assays using methods known in the art in light of this disclosure. For example, with regard to radical fringe-associated signal transduction, test agents can be applied to cells that express the radical fringe polypeptide and the fucose-specific glycosylation of the Notch receptor can be assessed (Moloney et al., Nature 406: 369-75, 2000). Preferably, the cells are genetically modified to overexpress the radical fringe polypeptide. Alternatively, radical fringe polypeptide, obtained from an endogenous or recombinant source, can be assayed *in vitro* for its glycosyltransferase activity (Moloney et al., *supra*). Preferably, the radical fringe polypeptide tested in the cell-based or *in vitro* assay is the human radical fringe. Alternatively, when using transgenic non-human mammals or keratinocytes expressing a radical fringe polypeptide under the control of a keratinocyte-specific, inducible regulatory sequence, agents can be tested for their ability to modulate keratinocyte proliferation and/or wound healing following the stimulus that induces expression of the transgene.

For assays to identify agents that modulate radical fringe gene expression, any type of cell that endogenously expresses the radical fringe gene can be used (Thelu et al., J. Invest. Dermatol. 111: 903-06, 1998; Moran et al., Mammalian Genome 10: 535-41, 1999; Egan et al., WO 98/17793). In wild type cells, expression of the radical fringe gene can be assessed at the level of radical fringe mRNA or polypeptide present in the cells. Alternatively, the cells can be genetically modified to express a transgene containing a radical fringe regulatory element operably linked to a coding sequence, preferably, the coding sequence for a reporter gene such as β-galactosidase.

The test agents used for screening may be selected individually or obtained from a compound library. Such libraries include biological libraries, peptoid libraries (libraries of molecules having the functions of peptides, but with novel, non-peptide backbones which are resistant to enzymatic degradation yet remain bioactive) (see, e.g., Zuckermann, J. Med. Chem. 37:2678-85, 1994), spatially addressable parallel solid phase or solution phase libraries, synthetic library methods requiring deconvolution, the "one-bead one-compound" library method, and synthetic library methods using affinity chromatography selection.

Examples of methods for the synthesis of molecular libraries can be found in the art, for example, in: DeWitt et al., Proc. Natl. Acad. Sci. (USA) 90:6909 (1993); Erd et al., Proc. Natl. Acad. Sci. (USA) 91: 11422, 1994; Zuckermann et al., J. Med. Chem., 37:2678,1994; Cho et al., Science, 261:1303, 1995; Carrell et al., Angew. Chem. Int. Ed. Engl. 33:2061, 1994; and in Gallop et al., J. Med. Chem. 37:1233, 1994.

Libraries of compounds may be presented in solution (e.g., Houghten, Biotechniques, 13:412-421, 1992), or on beads (Lam, Nature 354:82-841, 1991), on chips (Fodor, Nature 364:555-556, 1993), bacteria and spores (Ladner U.S. Patent No. 5,223,409), plasmids (Cull et al., Proc. Natl. Acad. Sci. USA. 89:1865-1869, 1992) or on phage (Scott et al., Science 249:386-390, 1990; Devlin, Science 249:404-406, 1990; Cwirla et al., Proc. Natl. Acad. Sci. (USA) 87:6378-6382, 1990; Felici, J. Mol. Biol. 222:301-310, 1991; Ladner, *supra*).

### The Nucleotide Coding Sequence and Amino Acid Sequence for the Human Radical Fringe Polypeptide

The invention encompasses the human radical fringe polypeptide sequence, for example, as shown in Fig. 1 B, and any variant, homologue, fragment, or derivative thereof.

The invention is also directed to nucleotide coding sequences that encode a human radical fringe polypeptide, for example, as shown in Fig. 1A, or any human variant, homologue, derivative, or fragment thereof. Also encompassed by the invention are human polynucleotide sequences that are capable of hybridizing to the polynucleotide sequences of the invention under various conditions of moderate or high stringency (see, e.g., Wahl and Berger, Methods Enzymol. 152: 399-407, 1987; and Kimmel, Methods Enzymol. 152: 507-11, 1987).

The nucleic acid sequences encoding the human radical fringe polypeptide may be extended utilizing a partial nucleotide sequence and employing various PCR-based methods known in the art to detect upstream sequences, such as promoters and regulatory elements. For example, one method which may be employed, restriction-site PCR, uses universal and nested primers to amplify unknown sequence from genomic DNA within a cloning vector (see, e.g., Sarkar, PCR Methods Applic. 2: 318-322, 1993.) Another method, inverse PCR, uses primers that extend in divergent directions to amplify unknown sequence from a circularized template. The template is derived from restriction fragments comprising a known genomic locus and surrounding sequences (see, e.g., Triglia et al., Nucleic Acids Res. 16: 8186, 1988). A third method, capture PCR, involves PCR amplification of DNA fragments adjacent to known sequences in human and yeast artificial chromosome DNA (see, e.g., Lagerstrom et al., PCR Methods Applic. 1: 111-119, 1991). In this method, multiple restriction enzyme digestions and ligations may be used to insert an engineered double-stranded sequence into a region of unknown sequence before performing PCR.

In another embodiment of the invention, a polynucleotide of the invention may be cloned in recombinant DNA molecules that direct expression of the human radical fringe polypeptide in appropriate host cells. The nucleotide sequences of the present invention can be engineered using methods generally known in the art in order to alter radical fringe-encoding sequences for a variety of purposes including, but not limited to, modification of the cloning, processing, and/or expression of the gene product.

DNA shuffling by random fragmentation and PCR reassembly of gene fragments and synthetic oligonucleotides may be used to engineer the nucleotide sequences. For example, oligonucleotide-mediated site-directed mutagenesis may be used to introduce mutations that create new restriction sites, alter glycosylation patterns, change codon preference, produce splice variants, and so forth. In another embodiment, sequences encoding a human radical fringe polypeptide may be synthesized, in whole or in part, using chemical methods well known in the art (see, e.g., Caruthers et al., Nucleic Acids Symp. Ser. 7: 215-223, 1980; and Horn et al., Nucleic Acids Symp. Ser. 7: 225-232, 1980). Alternatively, the human radical fringe polypeptide itself or a fragment thereof may be synthesized using chemical methods. For example, peptide synthesis can be performed using various solid- phase techniques (see, e.g., Roberge et al., Science 269: 202-204, 1995). Automated synthesis may be achieved using the ABI 431 A peptide synthesizer (Perkin-Elmer, Norwalk, CT). Additionally, the amino acid sequence of human radical fringe, or any part thereof, may be altered during direct synthesis and/or combined with sequences from other proteins, or any part thereof, to produce a variant polypeptide. The peptide may be substantially purified by preparative high performance liquid chromatography (see, e.g., Chiez and Regnier, Methods Enzymol. 182: 392-421, 1990). The composition of the synthetic peptides may be confirmed by amino acid analysis or by sequencing (see, e.g., Creighton, *Proteins, Structures and Molecular Properties,* WH Freeman, New York, NY 1984).

### Expression Vectors and Host Cells

In order to express a biologically active human radical fringe polypeptide, the nucleotide sequences encoding the human radical fringe polypeptide may be inserted into an appropriate expression vector, i.e., a vector which contains the necessary elements for transcriptional and translational control of the inserted coding sequence in a suitable host. These elements include regulatory sequences, such as enhancers, constitutive and inducible promoters, and 5' and 3' untranslated regions derived from the vector and/or from the polynucleotide sequences encoding a human radical fringe polypeptide. Such elements may vary in their strength and specificity. Specific initiation signals may also be used to achieve more efficient translation of sequences encoding radical fringe polypeptide. Such signals include the ATG initiation codon and adjacent sequences, e.g. the Kozak sequence. In cases where sequences encoding a human radical fringe polypeptide and its initiation codon and upstream regulatory sequences are inserted into the appropriate expression vector, no additional transcriptional or translational control signals may be needed. However, in cases where only coding sequence, or a fragment thereof, is inserted, exogenous translational control signals including an in-frame ATG initiation codon should be provided by the vector. Exogenous translational elements and initiation codons may be of various origins, both natural and synthetic. The efficiency of expression may be enhanced by the inclusion of enhancers appropriate for the particular host cell system used (see, e.g., Scharf et al., Results Probl. Cell Differ. 20: 125-162, 1994). Methods which are well known to those skilled in the art may be used to construct expression vectors containing sequences encoding a radical fringe polypeptide and appropriate transcriptional and translational control elements. These methods include *in vitro* recombinant DNA techniques, synthetic techniques, and *in vivo* genetic recombination (see, e.g., Sambrook et al., *Molecular Cloning, Laboratory Manual,* Cold Spring Harbor Press, Plainview NY, ch. 4, 8, and 16-17, 1989; Ausubel et al., *Current Protocols in Molecular Biology,* John Wiley & Sons, New York, NY, chs. 9, 13, and 16, 1995). A variety of expression vector/host systems may be utilized to contain and express sequences encoding a human radical fringe polypeptide. These include, but are not limited to, microorganisms such as bacteria transformed with recombinant bacteriophage, plasmid, or cosmid DNA expression vectors; yeast transformed with yeast expression vectors; insect cell systems infected with viral expression vectors (e.g., baculovirus); plant cell systems transformed with viral expression vectors (e.g., cauliflower mosaic virus, CaNW, or tobacco mosaic virus, TMV), with bacterial expression vectors (e.g., Ti or pBR322 plasmids), or animal cell systems. The invention is not limited by the host cell employed.

In bacterial systems, a number of cloning and expression vectors may be selected depending upon the use intended for polynucleotide sequences encoding the human radical fringe polypeptide. For example, routine cloning, subcloning, and propagation of polynucleotide sequences encoding radical fringe polypeptide can be achieved using a multifunctional *E. coli* vector such as pBlueScript (Stratagene, La Jolla, CA) or pSport1 plasmid (Life Technologies, Gaithersburg, MD). Ligation of sequences encoding radical fringe polypeptide into the vector's multiple cloning site disrupts the lacZ gene, allowing a colorimetric screening procedure for identification of transformed bacteria containing recombinant molecules. In addition, these vectors may be useful for *in vitro* transcription, dideoxy sequencing, single strand rescue with helper phage, and creation of nested deletions in the cloned sequence (see, e.g., Van Heeke and Schuster, J. Biol. Chem. 264: 5503-5509, 1989). When large quantities of radical fringe polypeptide are needed, e.g., for the production of antibodies, vectors which direct high level expression of radical fringe polypeptide may be used. For example, vectors containing the strong, inducible T5 or T7 bacteriophage promoter may be used.

In yeast expression systems, a number of vectors containing constitutive or inducible promoters, such as alpha factor, alcohol oxidase, or PGH promoters, may be used, for example, in the yeast *Saccharomyces cerevisiae* or *Pichia pastoris.* In addition, such vectors direct either the secretion or intracellular retention of expressed proteins and enable integration of foreign sequences into the host genome for stable propagation (see, e.g., Ausubel, 1995; Bitter et al., Methods Enzymol. 153: 516-544, 1987; and Scorer et al., BioTechnology 12:181-184,1994). Plant systems may also be used for expression of the human radical fringe polypeptide. Transcription of sequences encoding radical fringe polypeptide may be driven by viral promoters, e.g., the 35S and 19S promoters of CaMV used alone or in combination with the omega leader sequence from TMV (Takamatsu, EMBO J. 6: 307-311, 1987). Alternatively, plant promoters such as the small subunit of RUBISCO or heat shock promoters may be used (see, e.g., Coruzzi et al., EMBO J. 3: 1671-1680, 1984; Broglie et al., Science 224: 838-843, 1984; and Winter et al., Results Probl. Cell Differ. 17: 85-105, 1991). These constructs can be introduced into plant cells by direct DNA transformation or pathogen-mediated transfection (see, e.g., *The McGraw Hill Yearbook of Science and Technology,* McGraw Hill, New York NY, pp. 191-196, 1992).

In mammalian cells, a number of viral-based expression systems may be utilized. In cases where an adenovirus is used as an expression vector, sequences encoding radical fringe polypeptide may be ligated into an adenovirus transcription/translation complex consisting of the late promoter and tripartite leader sequence. Insertion in a non-essential E1 or E3 region of the viral genome may be used to obtain infective virus that express human radical fringe in infected host cells (see, e.g., Logan and Shenk, Proc. Natl. Acad. Sci. USA 81: 3655-3659, 1984). In addition, transcription enhancers, such as the Rous sarcoma virus (RSV) enhancer, may be used to increase expression in mammalian host cells. SV40 or EBV-based vectors may also be used for high-level protein expression.

HACs, BACs, or YACs may also be employed to deliver larger fragments of DNA than can be contained in and expressed from a plasmid. For example, HACs of about 6 kb to 10 Mb are constructed and delivered via conventional delivery methods (liposomes, polycationic amino polymers, or vesicles) for therapeutic purposes (see, e.g., Harrington et al., Nat. Genet. 15: 345-355, 1997). For long term production of recombinant proteins in mammalian systems, stable expression of the human radical fringe in cell lines is preferred. For example, sequences encoding the human radical fringe polypeptide can be transformed into cell lines using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. The purpose of the selectable marker is to confer resistance to a selective agent, and its presence allows growth and recovery of cells which successfully express the introduced sequences. Resistant clones of stably transformed cells may be propagated using tissue culture techniques appropriate to the cell type.

Any number of selection systems may be used to recover transformed cell lines. These include, but are not limited to, the herpes simplex virus thymidine kinase and adenine phosphoribosyltransferase genes, for use in TK⁻, and APR⁻ cells, respectively (see, e.g., Wigler et al., Cell 11: 223-232, 1997; Lowy et al., Cell 22: 817-823, 1980). Also, antimetabolite, antibiotic, or herbicide resistance can be used as the basis for selection. For example, Dhfr confers resistance to methotrexate; Neo confers resistance to the aminoglycosides neomycin and G-418; and Als and Pat confer resistance to chlorsulfuron and phosphinotricin acetyltransferase, respectively (see, e.g., Wigler et al., Proc. Natl. Acad. Sci. USA 77: 3567-3570, 1980; Colbere-Garapin et al., J. Mol. Biol. 150: 1-14, 1981). Additional selectable genes have been described, e.g., TrpB and HisD, which alter cellular requirements for metabolites (see, e.g., Hartman and Mulligan, Proc. Natl. Acad. Sci. USA 85: 8047-8051, 1988). Visible markers, e.g., anthocyanins, green fluorescent proteins (GFP; Clontech, Palo Alto, CA), β-glucuronidase and its substrate β-glucuronide, or luciferase and its substrate luciferin may be used. These markers can be used not only to identify transformants, but also to quantify the amount of transient or stable protein expression attributable to a specific vector system (see, e.g., Rhodes, Methods Mol. Biol. 55: 121-131, 1995). Although the presence/absence of marker gene expression suggests that the gene of interest is also present, the presence and expression of the gene may need to be confirmed. For example, if the sequence encoding the human radical fringe polypeptide is inserted within a marker gene sequence, transformed cells containing sequences encoding radical fringe polypeptide can be identified by the absence of marker gene function. Alternatively, a marker gene can be placed in tandem with a sequence encoding radical fringe polypeptide under the control of a single promoter. Expression of the marker gene in response to induction or selection usually indicates expression of the tandem gene as well.

In general, host cells that contain the nucleic acid sequence encoding the human radical fringe polypeptide and that express the human radical fringe polypeptide may be identified by a variety of procedures known to those of skill in the art. These procedures include, but are not limited to, DNA-DNA or DNA-RNA hybridizations, PCR amplification, and protein bioassay or immunoassay techniques which include membrane, solution, or chip based technologies for the detection and/or quantification of nucleic acid or protein sequences.

Immunological methods for detecting and measuring the expression of radical fringe using either specific polyclonal or monoclonal antibodies are known in the art. Examples of such techniques include enzyme-linked immunosorbent assays (ELISAs), radioimmunoassays (RIAs), and fluorescence activated cell sorting (FACS). A two-site, monoclonal-based immunoassay utilizing monoclonal antibodies reactive to two non-interfering epitopes on radical fringe polypeptide is preferred, but a competitive binding assay may be employed. These and other assays are well known in the art (see, e.g., Hampton, *Serological Methods, A Laboratory Manual,* APS Press, St. Paul, MN, Sect. IV, 1990; Coligan et al., *Current Protocols in Immunology,* Greene Pub. Associates and Wiley-Interscience, New York NY, 1997; and Pound, *Immunochemical Protocols,* Humana Press, Totowa, NJ, 1998). A wide variety of labels and conjugation techniques are known by those skilled in the art and may be used in various nucleic acid and amino acid assays.

Means for producing labelled hybridization or PCR probes for detecting sequences related to polynucleotides encoding the human radical fringe polypeptide include oligolabeling, nick translation, end-labeling, or PCR amplification using a labelled nucleotide.

Alternatively, the sequences encoding the human radical fringe polypeptide, or any fragments thereof, may be cloned into a vector for the production of an mRNA probe. Such vectors are known in the art, are commercially available, and may be used to synthesize RNA probes *in vitro* by addition of an appropriate RNA polymerase such as T7, T3, or SP6 and labelled nucleotides. These procedures may be conducted using a variety of commercially available kits (e.g., Amersham Pharmacia Biotech, Piscataway, NJ, Promega, Madison, WI, and US Biochemical, Cleveland OH). Suitable reporter molecules or labels which may be used for ease of detection include radionuclides, enzymes, fluorescent, chemiluminescent, or chromogenic agents, as well as substrates, cofactors, inhibitors, magnetic particles, and the like.

Host cells transformed with nucleotide sequences encoding radical fringe polypeptide may be cultured under conditions suitable for the expression and recovery of the protein from cell culture. The protein produced by a transformed cell may be secreted or retained intracellularly depending on the sequence and/or the vector used. As will be understood by those of skill in the art, expression vectors containing polynucleotides which encode the human radical fringe polypeptide may be designed to contain signal sequences which direct secretion of the human radical fringe polypeptide through a prokaryotic or eukaryotic cell membrane. In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Posttranslational processing which cleaves a "prepro" or "pro" form of the protein may also be used to specify protein targeting, folding, and/or activity.

Different host cells which have specific cellular machinery and characteristic mechanisms for post-translational activities (e.g., CHO, HeLa, MDCK, HEK293, and W138) are available from the American Type Culture Collection (ATCC, Manassas, VA) and may be chosen to ensure the correct modification and processing of the foreign protein. In another embodiment of the invention, natural, modified, or recombinant nucleic acid sequences encoding radical fringe polypeptide may be ligated to a heterologous sequence resulting in translation of a fusion protein in any of the aforementioned host systems. For example, a chimeric radical fringe protein containing a heterologous moiety that can be recognized by a commercially available antibody may facilitate the screening of peptide libraries for modulators of radical fringe polypeptide activity. Heterologous protein and peptide moieties may also facilitate purification of fusion proteins using commercially available affinity matrices. Such moieties include, but are not limited to, glutathione S-transferase (GST), maltose binding protein (MBP), thioredoxin (Trx), calmodulin binding peptide (CBP), 6-His, FLAG, c-myc, and hemagglutinin (HA). GST, MBP, Trx, CBP, and 6-His enable purification of their cognate fusion proteins on immobilized glutathione, maltose, phenylarsine oxide, calmodulin, and metal-chelate resins, respectively. FLAG, c-myc, and hemagglutinin (HA) enable immunoaffinity purification of fusion proteins using commercially available monoclonal and polyclonal antibodies that specifically recognize these epitope tags. A fusion protein may also be engineered to contain a proteolytic cleavage site located between the radical fringe polypeptide encoding sequence and the heterologous protein sequence, so that radical fringe polypeptide may be cleaved away from the heterologous moiety following purification. Methods for fusion protein expression and purification are discussed in Ausubel (1995, *supra,* ch. 10). A variety of commercially available kits may also be used to facilitate expression and purification of fusion proteins.

In a further embodiment of the invention, synthesis of a radiolabelled human radical fringe polypeptide may be achieved *in vitro* using the TNT rabbit reticulocyte lysate or wheat germ extract system (Promega). These systems couple transcription and translation of protein-coding sequences operably associated with the T7, T3, or SP6 promoters. Translation takes place in the presence of a radiolabelled amino acid precursor, for example, ³⁵S-methionine.

Fragments of the human radical fringe polypeptide may be produced not only by recombinant means, but also by direct peptide synthesis using solid-phase techniques (see, e.g., Creighton, *supra,* pp. 55-60). Protein synthesis may be performed by manual techniques or by automation. Automated synthesis may be achieved, for example, using the ABI® 431 A peptide synthesizer (Perkin-Elmer). Various fragments of the human radical fringe polypeptide may be synthesized separately and then combined to produce the full length molecule.

### Example 1

The transgenic mice were designed to express the murine radical fringe protein (Genbank U94350) under the control of a 2.4 kb fragment of the bovine Keratin 6 (K6) promoter obtained from Dr. Jose Jorcano (CIEMAT, Madrid, Spain) (see also, Ramirez et al., DNA and Cell Biol. 17: 177-85, 1998).

A pSE280 plasmid (Invitrogen, Carlsbad, CA) was used as the cassette backbone. In addition to a 2.4 kb K6 promoter, and the murine radical fringe cDNA, the cassette also contained the modified SV40t intron and a polyadenylation signal.

The transgene cassette was microinjected into a fertilized egg (B6SJLxC57BI6/J, Jackson Laboratories, Bar Harbor, ME) and implanted into the oviducts of pseudopregnant foster mothers (ICR strain, Taconic Laboratories, Germantown, NY).

DNA was collected from the offspring to confirm transgenic expression using PCR and Southern analysis. The PCR primers targeted the SV40 intron region of the transgene cassette. Transgenic expression produced no embryonic lethality in heterozygotes and no effects on fertility.

Transgene expression was assessed in the mice after induction with PMA. PMA (0.5 µg) was applied to the ears of the mice to stimulate hyperproliferation of keratinocytes on days 1, 4, and 7, and the mice were sacrificed on day 8. Total RNA was isolated from the inflamed ears for quantitative RT-PCR analysis to select the highest expressing lines. This analysis used PCR primers targeted to the SV40 intron region of the transgene and a fluorescently tagged probe. The two lines selected were 32645 and 32654, which expressed radical fringe protein at approximately 70 and 40 times, respectively, the level observed in the lowest expressing transgenic line.

The effect of injury-induced radical fringe transgenic expression was studied using a puncture wound bioassay and PMA application.

For the puncture wound assay, standardized 4 mm excisions of full skin thickness were created. These wounds were made in the dorsal skin of anaesthetised mice on day 1 and day 5 using a disposable skin biopsy trephine (Reed et al., Mech. Aging Dev. 89: 21-43, 1996). Daily caliper measurements were made to determine wound area (i.e., based upon the maximum distance between wound edges measured parallel and orthogonal to the midline). Wound healing was calculated as the percentage reduction in wound area with time. The animals were sacrificed on day 10. Skin samples (10 mm²) surrounding the two wounds and containing wounded skin, transitional skin, and control (non-wounded skin), were fixed in 10% formalin, stained, and analyzed histologically (Pathology Associates International, Frederick, MD). The following parameters were measured: epithelial growth, as estimated by measures of wound size in haematoxylin and eosin (Sigma Chemical, St. Louis, MO) stained sections; granulation bed collagen matrix deposition, as measured in Masson's Trichrome (*The Microtomist's Formulary and Guide,* Robert E. Kreuger Publishing Co., Inc., Huntington, NY, 1975) stained sections; and granulation bed angiogenesis, as estimated by morphometric analysis of the area occupied by red blood cells in haematoxylin and eosin stained sections.

In order to study the influence of radical fringe expression on the skin inflammatory response separately from the skin repair response, each mouse also received PMA (Sigma Chemical) ear treatment on days 3, 6, and 9. PMA promotes keratinocyte proliferation and causes aseptic inflammation of treated skin. The inflammation is initiated by the keratinocytes, and involves increased vascular permeability, epidermal hyperproliferation, and the recruitment of leukocyte infiltrate (Groves et al., J. Clin. Invest. 98: 336, 1996). PMA was topically applied (0.5 µg PMA in 100% ethanol) to each side of one ear. The other ear was treated with 100% ethanol as the control.

Ear thickness was measured by micrometer before the first application of PMA and 24 hours after each application. On day 10, ear skin was harvested, fixed in 10% buffered formal saline, sectioned transversely, and stained with haemotoxylin and eosin for histological analysis.

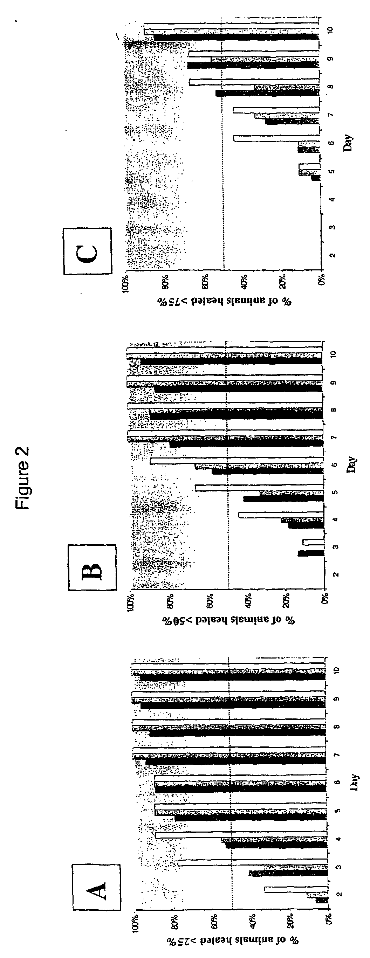

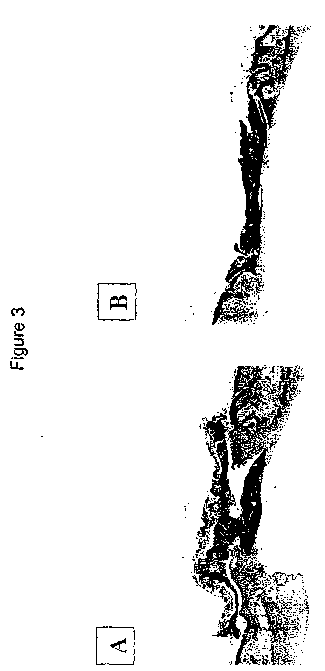

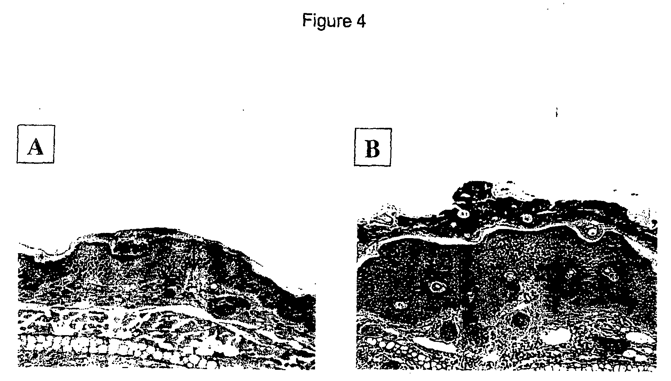

As compared to the wild-type controls, the transgenic radical fringe mice exhibited accelerated wound healing following punch biopsy as measured by the percent of animals healed at each day following injury (Fig. 2 A-C). The transgenic lines also exhibited an increased rate of reepithelialization in 5 day old wounds (Figure 3). In response to PMA treatment, the transgenic mice exhibited an increased keratinocyte proliferation, as demonstrated by an increased thickness in the keratinocyte layer of the skin (Figure 4).

### Example 2

Total RNA was extracted from 1 x 10⁸ human keratinocytes (Clonetics, San Diego, CA), using an RNeasy® Maxi kit (Qiagen, Valencia, CA), according to manufacturer's recommendations. Human keratinocyte total RNA (5 µg) was reverse transcribed to cDNA using ThermoScript-RT® (Invitrogen) according to manufacturer's recommendations, utilizing human forward and reverse primers as follows:

Forward 5'- ATG AGC CGC GCG CGT GGG GCG CTG TG -3' (SEQ ID NO: 3)

Reverse 5'- TCA CCG AGA GGT CGG GGC GCC CTG TT -3' (SEQ ID NO: 4).

The conditions for PCR amplification of the human radical fringe coding region were as follows: each 50 µl PCR sample contained 1 µl forward primer and 1 µl reverse primer solution (final concentration 1 µM); 2 µl keratinocyte cDNA, 1 µl dNTPs, 0.5 µl Platinum Taq Polymerase (Invitrogen); 5 µl 10X PCR buffer; 5 µl DMSO; 1.5 MgSO₄; 10 µl enhancer; and 23 µl dH₂O. The PCR reaction cycle comprised 30 cycles of 94°C for 1 min., 60°C for 1.5 min., and 68°C for 3 min., followed by 68°C for 10 min. and a 4°C soak.

The human radical fringe PCR product was cloned directly into the vector pCR II-TOPO vector using the TOPO® cloning system (Invitrogen) according to the manufacturer's instructions. The resulting insert was subsequently sequence-verified on both strands using ABI® DNA sequencing methodology as per the manufacturer's protocol (Perkin-Elmer). The nucleotide coding sequence for the human radical fringe gene is shown in Fig. 1A. The predicted amino acid sequence for the human radical fringe polypeptide is shown in Fig. 1 B.

## Claims

1. A transgenic non-human mammal expressing or capable of expressing a radical fringe transgene comprising a radical fringe coding sequence, wherein expression of said coding sequence in said mammal is driven by an operably linked regulatory sequence that directs inducible and keratinocyte-specific expression, and wherein said mammal exhibits accelerated wound healing and/or increased keratinocyte proliferation following the induction of transgene expression.

2. The transgenic non-human mammal of claim 1, wherein said regulatory sequence comprises the 2.4 kb fragment of the keratin 6 (K6) promoter.

3. The transgenic non-human mammal of claim 1, wherein said mammal is a rodent.

4. The transgenic non-human mammal of claim 3, wherein said rodent is a mouse.

5. The transgenic non-human mammal of claim 1, wherein said radical fringe transgene comprises the murine radical fringe coding sequence disclosed in Genbank Accession No. U94350.

6. The transgenic non-human mammal of claim 1, wherein said radical fringe transgene comprises the human radical fringe coding sequence of SEQ ID NO: 1.

7. A transgenic keratinocyte cell expressing or capable of expressing a radical fringe transgene, wherein said transgene comprises a radical fringe coding sequence, wherein expression of said coding sequence in said keratinocyte is driven by an operably linked regulatory sequence that directs inducible and keratinocyte-specific expression, and wherein said keratinocyte exhibits increased keratinocyte proliferation following the induction of transgene expression.

8. A transgenic ES cell comprising a radical fringe transgene, wherein said transgene comprises a radical fringe coding sequence, wherein expression of said coding sequence is driven by an operably linked regulatory sequence that directs inducible and keratinocyte-specific expression.

9. The transgenic cell of claim 7 or 8, wherein said regulatory sequence. comprises the 2.4 kb fragment of the keratin 6 (K6) promoter.

10. The transgenic cell of claim 7, 8 or 9, wherein said radical fringe transgene comprises the murine radical fringe coding sequence disclosed in Genbank Accession No. U94350.

11. The transgenic cell of claim 7, 8 or 9, wherein said radical fringe transgene comprises the human radical fringe coding sequence of SEQ ID NO: 1.

12. An isolated or purified human radical fringe polypeptide comprising the amino acid sequence of SEQ ID NO: 2, or an amino acid sequence encoded by a polynucleotide which will hybridize under highly stringent conditions to the full length complement of SEQ ID NO: 1.

13. An isolated or purified polypeptide comprising an amino acid sequence having at least 95% identity to an amino acid sequence comprising SEQ ID NO: 2.

14. An isolated or purified polynucleotide comprising a nucleic acid sequence encoding a polypeptide according to claim 12, the coding sequence shown in SEQ ID NO: 1, or a nucleic acid sequence which hybridizes to the full length of the complement of SEQ ID NO: 1 under highly stringent conditions.

15. A vector comprising the polynucleotide of claim 14.

16. A transgenic host cell comprising the polynucleotide of claim 14 operably linked to a regulatory sequence that drives expression in said host cell or a cell derived from said host cell.

17. A method for producing a polypeptide of claim 12, said method comprising culturing a transgenic host cell under conditions suitable for expression of the polypeptide, wherein said cell comprises the polynucleotide of claim 14 operably linked to a regulatory sequence that drives expression in said host cell.

18. A method of treating or preventing psoriasis in a patient, said method comprising administering an agent that reduces radical fringe activity in the epidermis of said patient.

19. A method of promoting wound healing, said method comprising administering an agent that increases radical fringe activity in the epidermis of said patient.

20. A method of identifying an agent that modulates radical fringe activity, said method comprising contacting an agent with the human radical fringe polypeptide of claim 12, and measuring radical fringe activity, wherein a difference between said radical fringe activity in the presence of the agent and in the absence of the agent is indicative that the agent modulates said activity.

21. A method of identifying an agent that modulates radical fringe activity, said method comprising contacting an agent with the transgenic host cell of claim 16, and measuring radical fringe activity, wherein a difference between said radical fringe activity in the presence of the agent and in the absence of the agent is indicative that the agent modulates said activity.

22. A method of identifying an agent that modulates radical fringe activity, said method comprising inducing transgene expression in the transgenic non-human mammal of claim 1 or the transgenic keratinocyte cell of claim 7, contacting said agent with said mammal or cell, and measuring radical fringe activity, wherein a difference in said radical fringe activity in the presence of the agent and in the absence of the agent is indicative that the agent modulates said activity.

23. An agent identifiable by the method of any one of claims 20, 21, or 22.

24. Use of an agent that inhibits radical fringe activity in the manufacture of a medicament for the treatment of patients with psoriasis or the prevention of psoriasis in patients.

25. Use of an agent that increases radical fringe activity in the manufacture of a medicament for the treatment of a wound.

26. Use as claimed in claim 25, wherein the wound is an acute wound, age-impaired wound, chronic ulcer, ulcer caused by venous stasis, ischemic ulcer, diabetic neuropathic ulcer, or pressure sore.

27. Use of a vector according to claim 15 for the manufacture of a medicament for the treatment of a wound.


SEQUENCE LISTING

<110> Pfizer Inc (CA, EP excluding UK, JP, US)
      Pfizer Ltd (EP(UK))

<120> Transgenic non-human mammal

<130> PCS 11087

<150> GB 0117392.1
<151> 2002-07-17

<150> US 60/306973
<151> 2002-07-19

<160> 4

<170> FastSEQ for Windows Version 4.0

<210> 1
<211> 996
<212> DNA
<213> Homo sapiens

<400> 1
atgagccgcg cgcgtggggc gctgtgccgg gcctgcctcg cgctggccgc ggccctggcc 60
gcgctgctgt tactgccgct gccgctgccc cgcgcgcccg ccccggcccg gacccccgcc 120
ccggccccgc gcgcgccccc gtcccggccc gctgccccca gcctgcggcc tgacgacgtc 180
ttcatcgccg tcaagaccac ccggaagaac cacgggccgc gcctgcggct gctgctgcgc 240
acctggatct cccgggcccg ccagcagacg tttatcttca ccgacgggga cgaccctgag 300
ctcgagctcc agggcggcga ccgtgtcatc aacaccaact gctcggcggt gcgcactcgt 360
caggccctct gctgcaagat gtccgtggag tatgacaagt tcattgagtc cgggcgcaag 420
tggttttgcc acgtggatga tgacaattat gtgaacgcca ggagcctcct gcacctgctc 480
tccagcttct cacccagcca ggacgtctac ctggggcggc ccagcctgga ccaccccatt 540
gaggccaccg agagggtcca gggtggcaga actgtgacca cggtcaagtt ctggtttgct 600
actggtgggg ccgggttctg cctcagcaga ggccttgccc tcaagatgag cccatgggcc 660
agcctgggca gcttcatgag cacagctgag caggtgcggc tgccggatga ctgcacagtt 720
ggctacatcg tggaggggct cctgggcgcc cgcctgctgc acagccccct cttccactct 780
cacctggaga acctgcagag gctgccgccc gacaccctgc tccagcaggt taccttgagc 840
catgggggtc ctgagaaccc acataacgtg gtgaacgtgg ctggaggctt cagcctgcat 900
caagacccca cacggtttaa gtctatccat tgtcttctgt acccagacac ggactggtgt 960
cccaggcaga aacagggcgc cccgacctct cggtga 996

<210> 2
<211> 331
<212> PRT
<213> Homo sapiens

<400> 2
Met Ser Arg Ala Arg Gly Ala Leu Cys Arg Ala Cys Leu Ala Leu Ala
 1               5                  10                  15
Ala Ala Leu Ala Ala Leu Leu Leu Leu Pro Leu Pro Leu Pro Arg Ala
            20                  25                  30
Pro Ala Pro Ala Arg Thr Pro Ala Pro Ala Pro Arg Ala Pro Pro Ser
        35                  40                  45
Arg Pro Ala Ala Pro Ser Leu Arg Pro Asp Asp Val Phe Ile Ala Val
    50                  55                  60
Lys Thr Thr Arg Lys Asn His Gly Pro Arg Leu Arg Leu Leu Leu Arg
65                  70                  75                  80
Thr Trp Ile Ser Arg Ala Arg Gln Gln Thr Phe Ile Phe Thr Asp Gly
                85                  90                  95
Asp Asp Pro Glu Leu Glu Leu Gln Gly Gly Asp Arg Val Ile Asn Thr
            100                 105                 110
Asn Cys Ser Ala Val Arg Thr Arg Gln Ala Leu Cys Cys Lys Met Ser
        115                 120                 125
Val Glu Tyr Asp Lys Phe Ile Glu Ser Gly Arg Lys Trp Phe Cys His
    130                 135                 140
Val Asp Asp Asp Asn Tyr Val Asn Ala Arg Ser Leu Leu His Leu Leu
145                 150                 155                 160

Ser Ser Phe Ser Pro Ser Gln Asp Val Tyr Leu Gly Arg Pro Ser Leu
                165                 170                 175
Asp His Pro Ile Glu Ala Thr Glu Arg Val Gln Gly Gly Arg Thr Val
            180                 185                 190
Thr Thr Val Lys Phe Trp Phe Ala Thr Gly Gly Ala Gly Phe Cys Leu
        195                 200                 205
Ser Arg Gly Leu Ala Leu Lys Met Ser Pro Trp Ala Ser Leu Gly Ser
    210                 215                 220
Phe Met Ser Thr Ala Glu Gln Val Arg Leu Pro Asp Asp Cys Thr Val
225                 230                 235                 240
Gly Tyr Ile Val Glu Gly Leu Leu Gly Ala Arg Leu Leu His Ser Pro
                245                 250                 255
Leu Phe His Ser His Leu Glu Asn Leu Gln Arg Leu Pro Pro Asp Thr
            260                 265                 270
Leu Leu Gln Gln Val Thr Leu Ser His Gly Gly Pro Glu Asn Pro His
        275                 280                 285
Asn Val Val Asn Val Ala Gly Gly Phe Ser Leu His Gln Asp Pro Thr
    290                 295                 300
Arg Phe Lys Ser Ile His Cys Leu Leu Tyr Pro Asp Thr Asp Trp Cys
305                 310                 315                 320
Pro Arg Gln Lys Gln Gly Ala Pro Thr Ser Arg
                325                 330

<210> 3
<211> 26
<212> DNA
<213> Homo sapiens

<400> 3
atgagccgcg cgcgtggggc gctgtg                                          26

<210> 4
<211> 26
<212> DNA
<213> Homo sapiens

<400> 4
tcaccgagag gtcggggcgc cctgtt                                          26